# EUROPEAN PATENT SPECIFICATION

(11) **EP 1 069 912 B1**
(45) Date of publication and mention of the grant of the patent: **25.07.2007**
(21) Application number: 99916414.8
(22) Date of filing: 02.04.1999
(51) Int. Cl.: A61K 47/12, A61K 38/30, A61K 9/08

(54) **INJECTABLE IGF-FORMULATIONS CONTAINING SUCCINATE AS BUFFERING AGENT**
IGF-ENTHALTENDE INJIZIERBARE FORMULIERUNGEN ENTHALTEND SUCCINATE ALS PUFFERMITTEL
FORMULATIONS D'IGF INJECTABLES CONTENANT UN SUCCINATE EN QUALITE D'AGENT TAMPON

(30) Priority: 03.04.1998 US 80008 P
(43) Date of publication of application: 24.01.2001
(73) Proprietor: Novartis Vaccines and Diagnostics, Inc., Emeryville, CA 94608 (US)
(72) Inventor: SHIRLEY, Bret, A.-Chiron Corporation, Emeryville, CA 94662-8097 (US); HORA, Maninder, S.-Chiron Corporation, Emeryville, CA 94662-8097 (US)
(74) Representative: Duckworth, Timothy John
(86) International application number: PCT/US1999/007531
(87) International publication number: WO 1999/051272

(56) References cited:
- EP-A- 0 284 249
- WO-A-95/31213
- WO-A-96/40894
- JP-A- 9 304 379
- US-A- 5 151 265
- US-A- 5 681 814

## Description

### FIELD OF THE INVENTION

The field of the invention is injectable formulations of pharmaceutically active agents for *in vivo* use. The formulations to which the invention is directed are designed to minimize the pain associated with components in injectable formulations, other than active components. The invention is particularly directed to pharmaceutical formulations that are buffered with succinate and that provide for reduced pain upon injection. Human insulin-like growth factor I (hIGF-I) is the pharmaceutically active agent.

### BACKGROUND OF THE INVENTION

Numerous studies have shown that subcutaneous injection can be painful (Ipp et al. (1990) Pediatrics 85:134-135); Zindel (1989) Conn. Med. 53:741-744); Gazzaniga et al. (1993) Int. Surg. 78:271-275). Little information exists on the causes of pain on injection due to formulation variables. It has been postulated that factors causing pain include injection volume, speed of injection, osmolality, pH, injection site, size and quality of injection needle, presence of irritating substances, and temperature of the solution (Fransson et al. (1996) J. Pharm. Pharmacol. 48:1012-1015).

Most injectable pharmaceutical formulations contain a buffer to stabilize the pharmaceutically active agent against the chemical degradation that might occur if the pH changes appreciably. The most commonly used buffer systems in injectable pharmaceutical formulations are citrates, acetates and phosphates. However, the injection of such formulations has been associated with pain. For example, it has been reported that the injection of a saline solution is less painful than the injection of citrate buffer (Fleischaker et al. (1993) J. Clin. Pharmacol. 33:182-190).

Frenken et al. (1993) Am. J. Kidney Dis. 22:553-556 ) reported a clinical study in which the role of 20 mM citrate in causing pain after subcutaneous administration of Epoetin α was evaluated. The results suggested that 20 mM sodium citrate (pH 6.9) causes significant pain compared to saline. The results of another study by this same group suggested that (1) injection of smaller volumes was beneficial; (2) adjustment of Eprex osmolarity to bring it to isotonicity did not reduce pain; and (3) inclusion of benzyl-alcohol in the diluted injection lessened the pain, presumably due to anesthetic effects (Frenken et al. (1994) Nephrol. Dial. Transplant. 9:1295-1298).

The injection of IGF-I has been associated with pain. The literature teaches that buffers preferred in IGF-I formulations are acetate, phosphate and citrate buffers. For example, U.S. patent 5,374,620 mentions the possible use of a succinate buffer in pharmaceutical formulations containing IGF-I and GH. However, the '620 patent teaches that sodium acetate, optionally in combination with sodium citrate, is the preferred buffer.

Fransson et al. (1996) (J. Pharm. Pharmacol. 48:1012-1015) studied formulations for minimizing pain with subcutaneous injection of hIGF-I. The study evaluated how pH, buffer concentration, and presence of hIGF-I affect local tolerance to subcutaneous injection of the solution. The study reported that (1) a buffer with high buffering capacity (50 mM phosphate) at pH 6.0 made isotonic with NaCl caused significantly more pain than a 10 mM phosphate buffer formulation; (2) pH 7 phosphate buffered hIGF formulations were well-tolerated; (3) 10 mM phosphate, pH-based hIGF-I formulations were as well-tolerated as the pH 7 formulation; and (4) hIGF-I itself did not cause pain.

WO 94/15584 is directed to injectable formulations for subcutaneous administration of IGF-I designed to reduce pain. The reference teaches the use of a phosphate buffer to reduce the pain of subcutaneous injection.

EP-A-0 284 249 discloses a lyophilized lymphokine composition comprising a therapeutically effective amount of a lymphokine such as an interferon and, amongst other components, a buffer such as a combination of succinic acid and sodium succinate. US-5,151,265 discloses a liquid pharmaceutical composition comprising non-lyophilising gamma-interferon. The composition includes a buffer, which may be citrate, succinate, tartrate, fumarate, gluconate, oxalate, lactate or acetate and maintains the composition at a pH from 4.0 to 6.0. WO 96/40894 discloses compositions for the treatment of insulin resistance through inhibition of serine phosphorylation of protein kinase C. Such compositions may include a succinate buffer. US 5,681,814 discloses formulations of IGF1 comprising osmolyte, stabilizer, a buffered solution and optionally a surfactant. Examples of buffer include succinate.

WO 95/31213 discloses interferon-beta liquid formulations stabilized with a polyol, a non-reducing sugar or an amino acid. Compositions comprising succinate buffer are also disclosed. JP 09/304379 discloses prophylactic methods for reducing the risk of thrombosis which employ administration of TFPI (tissue factor pathway inhibitor) to achieve higher levels in plasma. The TFPI can be formulated according to any means known in the art, including formulations including buffers.

Subjects injected with IGF-I formulations buffered by phosphate or acetate report less pain than with a citrate formation. Nevertheless, a significant level of pain remains associated with the injection of IGF-I formulations buffered by phosphate or acetate. Accordingly, an object of the invention is to provide improved pharmaceutical compositions that result in reduced pain upon injection. A specific object of the invention is to provide a pharmaceutical composition that allows the injection of hIGF-I with reduced pain.

A further specific object of the invention is to provide a pharmaceutical composition in which the pharmaceutically active agent is stable and thus can be stored for extended periods of time without significant physical and/or biological breakdown.

### SUMMARY OF THE INVENTION

The present invention is based on the inventors' discovery that pharmaceutical formulations buffered with succinate cause less pain on injection than formulations containing more commonly used buffers, such as phosphate, acetate and citrate buffers. In particular, the inventors have discovered succinate buffers reduce the pain associated with the injection of IGF-I formulations. Based upon this discovery, it is now possible to develop pharmaceutical compositions for IGF-I and other pharmaceutically active agents with reduced pain due to injection. Heretofore, the medical literature did not suggest the use of succinate buffer to minimize the pain associated with injection.

The present invention provides an injectable pharmaceutical composition comprising human insulin-like growth factor 1 (IGF-I) or a biologically active variant thereof and a buffer, wherein said buffer consists substantially of succinate at a concentration of 10mM to 40 mM and a counterion, and wherein said variant is a polypeptide having IGF-I activity and at least 70% amino acid sequence identity to the amino acid sequence for human IGF-I.

Use of succinate as the buffer provides reduced pain upon injection. In a preferred embodiment, the composition of the invention comprises IGF-1 and sodium succinate.

The pharmaceutical compositions of the invention can be stored for extended periods of time while maintaining the physical and biological integrity of the pharmaceutically active agent. The invention is also directed to methods for administering any of the pharmaceutical compositions described above and herein.

### BRIEF DESCRIPTION OF THE DRAWINGS

Figure 1. Stability of rhIGF-I as a function of pH. In this study, citrate-phosphate buffer at a pH range of 4.0-7.0 was formulated with rhIGF. Percent main peak (peak that contains the native molecule) integrity was measured over a period of eight weeks at a temperature of 50°C. Measurement was by CN (cyano)-RP-HPLC.
Figure 2. Stability of rhIGF-I as a function of pH. In this study, citrate-phosphate buffer at a pH range of 4.0-7.0 was formulated with rhIGF. Percent mitogenic activity was measured over a period of eight weeks at a temperature of 50°C.
Figure 3. Effect of various buffer species at pH 6.0 and pH 6.5 on the stability of rhIGF. In this study, various buffers were formulated with rhIGF-I. Main peak integrity was measured over a period of eight weeks at a temperature of 50°C. The integrity was measured by CN-RP-HPLC.
Figure 4. Effect of various buffer species at pH 6.0 and pH 6.5 on the stability ofrhIGF. The measurement of percent monomer (native molecule) remaining was over a period of eight weeks at a temperature of50°C. The percent monomer remaining was measured by non-reducing SDS-PAGE.
Figure 5. Effect of sodium citrate and sodium succinate buffers at various concentrations on the stability of rhIGF-I. Stability was measured over a period of eight weeks at 50°C. Measurement was done by assaying the percent monomer by non-reducing SDS-PAGE.
Figure 6. Effect of sodium citrate and sodium succinate buffers at various concentrations on the stability of rhIGF-I. Stability was measured by assay of mitogenic activity over a period of eight weeks at 50°C.

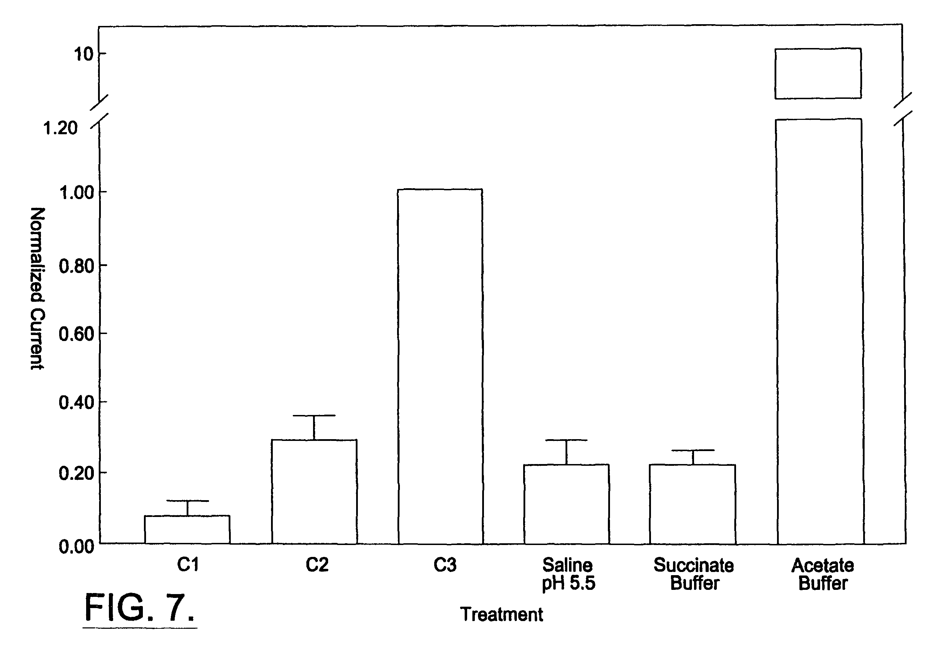

Figure 7. Data from a nociceptor activation model designed to predict the pain on injection induced by formulations. In this model, capsaicin (a compound known to cause pain on injection) (Santos et al. (1997) Neuropeptides 31:381-389) is used to generate a standard curve. Formulations were then tested and given a value based on the standard curve. All formulations were then compared to normal saline. Two buffers were tested and compared to normal saline: 87 mM acetic acid, 13 mM sodium acetate, pH 4.0; and 10 mM sodium succinate, 140 mM sodium chloride, pH 6.0. C1, C2 and C3 are different concentrations of capsaicin.

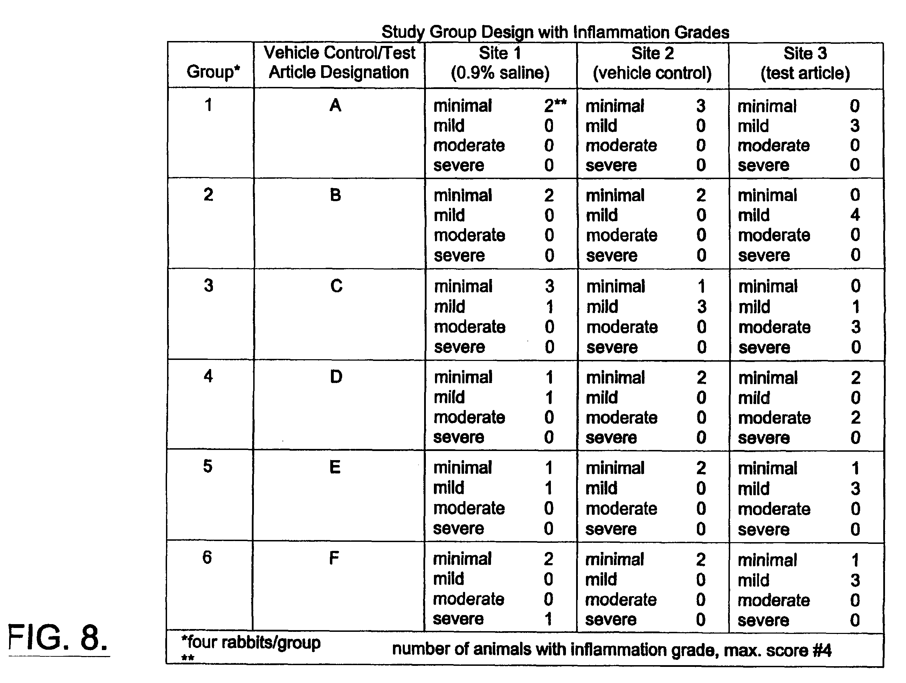

Figure 8. Local irritant effects of pharmaceutical formulations injected subcutaneously. Rabbits were injected with saline, a vehicle control (buffer without pharmaceutical agent), and a test article (buffer plus pharmaceutical agent) at three separate sites. A: 10 mM sodium succinate, 140 mM sodium chloride, pH 6.0; B: 10 mM sodium citrate, 135 mM sodium chloride, pH 6.0; C: 87 mM acetic acid, 13 mM sodium acetate, 50 mM sodium chloride; D: 1 mM methionine, 135 mM sodium chloride, pH 6.0; E: 10 mM sodium succinate; 125 mM arginine, 20 mM sodium chloride, pH 6.0; and F: 1.9% sucrose, 97 mM sodium chloride, pH 4.8.

### DETAILED DESCRIPTION OF THE INVENTION

The invention is directed to an injectable pharmaceutical composition comprising IGF-1 and a buffer, wherein said buffer consist substantially of a succinate buffer. Pharmaceutical formulations buffered with succinate cause less pain upon injection than similar formulations containing non-succinate buffers, such as citrate, phosphate or acetate buffers.

The invention further provides a composition of the invention for use in a method of treatment, prevention or diagnosis of a disease or condition, in a human or animal subject by injection.

By "succinate buffer" is meant buffer comprising a salt of succinic acid. In a preferred embodiment, the succinate cation is sodium. However, any cation is expected to be effective. Other possible succinate cations include potassium, ammonium, calcium and magnesium. In one embodiment, the pharmaceutically active agent IGF-1 provides the cation.

The pharmaceutical compositions of the invention are buffered substantially or essentially by a succinate buffer. It will be understood, however, that other compounds may be present in the compositions of the invention that have some relatively minor buffering capacity. Examples of such compounds are the pharmaceutically active agent itself, stabilizing agents, and the like.

The succinate buffer can be used at a range of concentrations from 10 mM up to 40 mM. Suitable concentration ranges include 11-30 mM; 12-25 mM; 13-20 mM; 14-19 mM; and about 15 mM. The preferred concentration ranges of succinate buffer are less than 40 mM, less than 35 mM, less than 30 mM, less than 25 mM, less than 20 mM, and less than 15 mM. Most preferably, the concentration- of the succinate buffer is about 10 mM.

Preferably the pharmaceutically active agent IGF-1 is present in the compositions of the invention at a concentration useful for the administration of a pharmaceutically effective amount of said agent to a subject. The pharmaceutically active agent may be prepared from any source, including purification from a mammal, recombinant production or chemical synthesis.

In the present invention, the pharmaceutically active agent is human IGF-I (hIGF-I) or a biologically active variant thereof. Most preferably, the pharmaceutically active agent is recombinant hIGF-I (rhIGF-I).

The term "IGF-I" as used herein refers to insulin-like growth factor I, in native form and biologically active variants. Suitable biologically active variants can be IGF-I fragments, analogues, and derivatives. Preferably the IGF-I is from the same species as the species undergoing treatment. However, the IGF-I of the present invention can be encoded by any animal species including, avian, canine, bovine, porcine, equine, and human.

IGF-I may be purified from a natural source, chemically synthesized or produced recombinantly. Human IGF-I has been purified from plasma and its complete amino acid sequence is established (Rinderknecht et al. (1978) J. Biol. Chem. 253:2769-2776). Sequences with extensive homologies to human IGF-I are present in IGF-I purified from the plasma of other species.

The compositions of the present invention may include biologically active variants of IGF-I. IGF-I variants differ from naturally occurring IGF-I molecules due to chemical modification or to amino acid insertions, deletions, substitutions (including chemically modified amino acids), and carboxy or amino terminal truncations or fusions. Such variants should substantially or completely retain IGF-I activities sufficient for the beneficial treatment of a given disorder. In particular, variants should retain the ability to bind to IGF-I receptor sites. IGF-I variants are known to those skilled in the art. See, for example, U.S. Patent No. 5,374,620.

By "substantially" is meant activity which may be quantitatively different, yet is qualitatively the same. When a protein has multiple activities, a variant can have substantially the same activity if it has less than all the activities, as long as one of these is retained. Preferably, the variant has at least the same activity as the native molecule.

IGF-I activity can be measured using standard IGF-I bioassays known to those skilled in the art. Representative assays include known radioreceptor assays using placental membranes (see, *e.g.,* U.S. Patent No. 5,324,639; Hall et al. (1974) J. Clin. Endocrinol. and Metab. 39:973-976; and Marshall et al. (1974) J. Clin. Endocrinol. and Metab. 39:283-292), a bioassay that measures the ability of the molecule to enhance incorporation of tritiated thymidine, in a dose-dependent manner, into the DNA of BALB/c 3T3 fibroblasts (see, *e.g*., Tamura et al. (1989) J. Biol. Chem. 262:5616-5621), herein incorporated by reference.

By "IGF-I fragment" is intended a peptide that is only a part of the intact IGF-I sequence and structure. It includes, but is not limited to, a C-terminal deletion or N-terminal deletion of IGF-I. The term "fragment" is meant to include any portion of the protein that provides a segment that substantially or completely retains the essential biological function(s) of the protein. Fragments may be made from any source, such as, for example, from naturally occurring peptide sequences, synthetic or chemically-synthesized peptide sequences, and genetically-engineered peptide sequences.

By "analog" is intended an analog of either IGF-I or an IGF-I fragment that comprises a native IGF-I sequence and structure having one or more amino acid substitutions, insertions, or deletions. An analog encompasses a protein that is the same or substantially similar in function to the native protein. For example, an analog of IGF-I protein is a protein that does not have the same amino acid sequence as the IGF-I protein but which is sufficiently homologous so as to substantially or completely retain the biological activity. Peptides having one or more peptoids (peptide mimics) are also encompassed by the term "analog" (see International Publication No. WO 91/04282).

Suitable modifications of IGF-I, IGF-I fragments, or their respective analogs, include, but are not limited to, glycosylation, phosphorylation, PEGylation, or other addition of foreign moieties, so long as the IGF-I activity is substantially or completely retained. Such modifications may improve the compound's solubility, absorption, biological half life, decrease the toxicity of the molecule, or eliminate or attenuate any undesirable side effect of the molecule, etc. Derivatives and specifically, chemical moieties capable of mediating such effects are disclosed in Remington's Pharmaceutical Sciences (1980). Procedures for coupling such moieties to a molecule are well known in the art.

IGF-I variants will generally have at least 70%, preferably 80%, more preferably 90% to 95% or more, and most preferably 98% or more amino acid sequence identity to the amino acid sequence of the reference IGF-I molecule. A variant may differ by as few as 10, as few as 5, as few as 4, 3, 2, or even 1 amino acid residue. By "sequence identity" is intended the same amino acid residues are found within the IGF-I variant and the reference IGF-I molecule when a specified, contiguous segment of the amino acid sequence of the variant is aligned and compared to the amino acid sequence of the reference molecule. Sequence identity is determined according to the Wisconsin Sequence Analysis Package, Version 8 GAP program (available from Genetics Computer Group, Madison, Wisconsin), using the default settings.

For purposes of optimal alignment of the two sequences, the contiguous segment of the amino acid sequence of the variant may have additional amino acid residues or deleted amino acid residues with respect to the amino acid sequence of the reference molecule. The contiguous segment used for comparison to the reference amino acid sequence will comprise at least twenty (20) contiguous nucleotides, and may be 30, 40, 50, 100, or more nucleotides. Corrections for increased sequence identity associated with inclusion of gaps in the variant's amino acid sequence can be made by assigning gap penalties. Methods of sequence alignment are well known in the art.

When considering percentage of amino acid sequence identity, some amino acid residue positions may differ as a result of conservative amino acid substitutions, which do not affect properties of protein function. In these instances, percent sequence identity may be adjusted upwards to account for the similarity in conservatively substituted amino acids. Such adjustments are well known in the art. See, for example, Meyers and Miller (1988) Computer Applic. Biol. Sci. 4:11-17.

The art provides substantial guidance regarding the preparation and use of such analogs, as discussed further below. A fragment of IGF-I will generally include at least about 10 contiguous amino acid residues of the full-length molecule, preferably about 15-25 contiguous amino acid residues of the full-length molecule, and most preferably about 20-50 or more contiguous amino acid residues of full-length IGF-I. In preparing the IGF-I variants, one of skill in the art can readily determine which modifications to the native protein nucleotide or amino acid sequence will result in a variant that enables preparation of the highly concentrated form of the IGF-I variant in accordance with the methods disclosed in the present invention. These will generally be conservative amino acid substitutions that preserve the charge of the substituted residue (*e.g*., aspartic acid for glutamic acid).

Several IGF-I analogs and fragments are known in the art and include those described in, for example, Proc. Natl. Acad. Sci. USA (1986) 83:4904-4907; Biochem. Biophys. Res. Commun. (1987) 149:398-404; J. Biol. Chem. (1988) 263:6233-6239; Biochem. Biophys. Res. Commun. (1989) 165:766-771; Forsbert et al. (1990) Biochem. J. 271:357-363; U.S. Patent Nos. 4,876,242 and 5,077,276; and International Publication Nos. WO 87/01038 and WO 89/05822. Representative analogs include one with a deletion of Glu-3 of the mature molecule, analogs with up to 5 amino acids truncated from the N-terminus, an analog with a truncation of the first 3 N-terminal amino acids (referred to as des(1-3)-IGF-I, des-IGF-1, tIGF-I, or brain IGF), and an analog including the first 17 amino acids of the B chain of human insulin in place of the first 16 amino acids of human IGF-I.

Methods for making IGF-I fragments, analogs, and derivatives are available in the art. See generally U.S. Patent Nos. 4,738,921, 5,158,875, and 5,077,276; International Publication Nos. WO 85/00831, WO 92/04363, WO 87/01038, and WO 89/65822; and European Patent Nos. EP 135094, EP 123228, and EP 128733.

IGF-I can be from any animal species including, but not limited to, avian, canine, bovine, porcine, equine, and human. Preferably the IGF-I is from a mammalian species when the treatment is of a mammalian IGF-I-responsive disorder, and more preferably is from a mammal of the same species as the mammal undergoing treatment for such a disorder. It is recognized that the IGF-I can be made by recombinant methods using the corresponding coding sequence for IGF-I from the animal species of interest. Such recombinant methods are discussed in more detail below.

Biologically active variants of IGF-I should retain IGF-I activities, particularly the ability to bind to IGF-I receptor sites. IGF-I activity may be measured using standard IGF-I bioassays. Representative assays include known radioreceptor assays using placental membranes (see, *e.g.,* U.S. Patent No. 5,324,639; Hall et al. (1974) J. Clin. Endocrinol. and Metab. 39:973-976; and Marshall et al. (1974) J. Clin. Endocrinol. and Metab. 39:283-292), a bioassay that measures the ability of the molecule to enhance incorporation of tritiated thymidine, in a dose-dependent manner, into the DNA of BALB/c 3T3 fibroblasts (see, *e.g.,* Tamura et al. (1989) J. Biol. Chem. 262:5616-5621), and the like; herein incorporated by reference. Preferably, the variant has at least the same activity as the native molecule.

The IGF-I used in making the compositions of the present invention can be in its substantially purified, native, recombinantly produced, or chemically synthesized forms. For example, the IGF-I can be isolated directly from blood, such as from serum or plasma, by known methods. See, for example, Phillips (1980) New Eng. J Med. 302:371-380; Svoboda et al. (1980) Biochemistry 19:790-797; Cornell and Boughdady (1982) Prep. Biochem. 12:57; Cornell and Boughdady (1984) Prep. Biochem. 14:123; European Patent No. EP 123,228; and U.S. Patent No. 4,769,361.

Alternatively, IGF-I can be synthesized chemically, by any of several techniques that are known to those skilled in the peptide art. See, for example, Li et al. (1983) Proc. Natl. Acad. Sci. USA 80:2216-2220, Stewart and Young (1984) Solid Phase Peptide Synthesis (Pierce Chemical Company, Rockford, Illinois), and Barany and Merrifield (1980) The Peptides: Analysis, Synthesis, Biology, ed. Gross and Meienhofer, Vol. 2 (Academic Press, New York, 1980), pp. 3-254, for solid phase peptide synthesis techniques; and Bodansky (1984) Principles of Peptide Synthesis (Springer-Verlag, Berlin); and Gross and Meienhofer, eds. (1980) The Peptides : Analysis, Synthesis, Biology, Vol. 1 (Academic Press, New York), for classical solution synthesis. IGF-I can also be chemically prepared by the method of simultaneous multiple peptide synthesis. See, for example, Houghten (1985) Proc. Natl. Acad. Sci. USA 82:5131-5135; and U.S. Patent No. 4,631,211.

Genetic engineering by recombinant DNA techniques can be the most efficient way of producing IGF-I. The human DNA sequence encoding IGF-I is known and can be introduced into host cells for expression. IGF-I can be produced by recombinant DNA techniques in *E*. *coli,* yeast, insect, and mammalian cells. Secreted IGF-I can be made by adding a signal sequence to the DNA sequence encoding IGF-I. In addition, the DNA sequence encoding IGF-I can be manipulated to make IGF-I fragments, analogues, or derivatives. Such recombinant DNA techniques are generally available in the art. See, for example, International Publication No. WO 96/07424, where recombinant human IGF-I protein is produced in yeast.

The appropriate concentration of IGF-I in the compositions of the invention depends upon the solubility in the specific buffer used and the desired therapeutic amount for the given dose. Typically, the concentration of IGF-I in the liquid compositions of the invention will range from 0.01-200 mg/ml. Preferably the concentration of IGF-I is 0.1-20.0 mg/ml, and more preferably 2.0-10.0 mg/ml.

The pharmaceutical composition comprising a IGF-I and a succinate buffer may also contain other components that facilitate IGF treatment. Such components include IGF-I binding proteins, IGF-I receptors, and the acid-labile subunit of the IGF binding complex. It is generally known that IGF-I action in cartilage is modulated by IGF-I binding proteins, at least six of which (IGFBP-1 through IGFBP-6) have been isolated (see Baxer et al. (1989) Prog. Growth Factors Res. 1:49-68; and Rechler et al. (1992) Growth Regul. 2:55-68). Of these, IGFBP-3 is the primary binding protein for IGF-I. Its presence may enhance the stimulatory effect of IGF-I on proteoglycan synthesis (see Chevalier et al. (1996) British J. Rheumat. 35:515-522). In addition, an acid labile glycoprotein also has been shown to be associated with the protein complex formed by IGF-I and its binding proteins. Thus, the pharmaceutical composition of the invention may contain such acid-labile glycoprotein and IGF-I binding proteins, when proven to facilitate the desired effect of IGF-I on cartilage maintenance and/or regeneration.

The amount of IGFBPs to be administered with IGF-I can be determined according to the molar ratio between IGF-I and IGFBPs. This molar ratio can range from 0.5:1 to 3:1, preferably 1:1 (see U.S. Patent No. 5,187,151). All such references to components facilitating IGF-promoted maintenance and/or regeneration of cartilage are herein incorporated by reference.

The IGF-I pharmaceutical composition in accordance with the present invention may further comprise one or more other therapeutic agents that are effective in treating other disorders in the individual, as long as the biochemical actions of the additional therapeutic agents do not interfere with the efficacy of intended action of the IGF-I treatment. Examples of such agents include, antibiotics, anti-inflammatory agents, and the like.

The pharmaceutical compositions of the invention may include one or more protease inhibitors. An exemplary protease inhibitor is sodium pentosan polysulfate (PPS), a polysulfated polysaccharide.

The compositions of the invention may optionally include stabilizing agents including, amino acids (such as arginine, lysine, and glycine), sugars (such as sucrose, mannitol, and trehalose), salts (such as NaCl and MgCl₂), surfactants, PEG, preservatives, antimicrobial agents, complexing agents (such as EDTA), and anti-oxidants. For hIGF-I, formulations can include low levels of methionine, such as 1 mM, to slow oxidation and preserve against aggregation or precipitation of the protein.

Preferably, the pharmaceutical composition is isotonic with the cells of the subject. Preferably the pharmaceutical composition is isotonic with the erythrocytes of a subject. More preferably, the pharmaceutical composition is isotonic with human erythrocytes. Thus, in one embodiment, the pharmaceutical composition contains a sufficient concentration of at least on tonicifying agent such that the composition is isotonic.

By "isotonic" is meant a solution in which a cell will neither shrink nor swell. An example of an isotonic solution is 0.9% sodium chloride in water. Typically, an isotonic solution will have about the same osmotic pressure as the fluid phase of a subject's cells or tissue. However, a solution that is isosmotic with intracellular fluid will not be isotonic if it contains a solute that freely permeates cell membranes. To determine is a solution is isotonic, it is necessary to identify the concentration of solutes at which cells will retain their normal size and shape. Methods of determining the isotonicity of a solution are known to those skilled in the art. See, for example, Setnikar et al. (1959) JAPhA Sci Ed 48:628.

In a preferred embodiment, the pharmaceutical composition further comprises sodium chloride. Sodium chloride is included to provide isotonicity to the formulation. Therefore, the concentration of sodium chloride in the formulation will depend upon the contribution of other components to tonicity. Preferably, the concentration of sodium chloride is about 140 mM.

Those skilled in the art are familiar with a variety of pharmaceutically acceptable solutes useful in providing isotonicity in pharmaceutical compositions. Thus, the compositions of the invention further encompass components that can be used to provide isotonicity, for example, sodium chloride, glycine, mannitol, glycerol, sucrose, and other carbohydrates, acetic acid, other organic acids or their salts, and relatively minor amounts of citrates or phosphates. The ordinary skilled person would know of additional agents that are suitable for providing optimal tonicity.

The pH of the pharmaceutical composition affects the both the level of pain upon injection and the stability of the pharmaceutically active ingredient. A given pharmaceutically active ingredient will have greatest stability in a certain pH range of a particular buffer. The optimal pH which provides the desired balance of stability and minimal or no pain upon injection may be determined by those skilled in the art. However, the pH range could encompass formulations of pH 4.0-7.5: Suitable pH's include 4.1, 4.2, 4.3, 4.4, 4.5, 4.6, 4.7, 4.8, 4.9, 5.0, 5.1, 5 .2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5. Suitable pH ranges are 4.5-7.2; 4.6-7.1 ; 4.7-7.0; 4.8-6.9 4.9-6.8, 5.0-6.7; 5.1-6.6; 5.2-6.5; 5.3-6.4; 5.4-6.3; 5.5-6.2; 5.7-6.1; and 5.8-6.0. A preferred range is pH 4.6-6.6. Most preferably, the pH is 5.6. For compositions containing IGF-I, the preferred pH is 6.0.

The pharmaceutical composition can be formulated as a solution, suspension, or emulsion. It can also be in the form of lyophilized powder, which can be converted into solution, suspension, or emulsion before administration. Storage can also be facilitated by adding proteins such as human albumin that can reduce the loss of activity of the pharmaceutically active protein. Thus, albumin could function as a stabilizing protein during the freeze-drying process. The methods for formulating a pharmaceutical composition are generally known in the art. A thorough discussion of formulation and selection of pharmaceutically acceptable carriers, stabilizers, and isomolytes can be found in Remington's Pharmaceutical Sciences (1990) (18th ed., Mack Pub. Co., Eaton, Pennsylvania).

The pharmaceutically active agent can also be formulated in a sustained-release form to prolong the presence of the pharmaceutically active agent in the treated mammal, generally for longer than one day. Many methods of preparation of a sustained-release formulation are known in the art and are disclosed in Remington's Pharmaceutical Sciences (1990) (18th ed., Mack Pub. Co., Eaton, Pennsylvania. Generally, the agent can be entrapped in semipermeable matrices of solid hydrophobic polymers. The matrices can be shaped into films or microcapsules.

Examples of such matrices include polyesters, copolymers of L-glutamic acid and gamma ethyl-L-glutamate (Sidman et al. (1983) Biopolymers 22:547-556), polyactides (U.S. Patent No. 3,773,919 and EP 58,481), polyactate polyglycolate (PLGA), hydrogels (see, for example, Langer et al. (1981) J. Biomed. Mater. Res. 15:167-277; Langer (1982) Chem. Tech. 12:98-105), non-degradable ethylene-vinyl acetate, degradable lactic acid-glycolic acid copolyers such as the Lupron Depot^{™}, and poly-D-(-)-3-hydroxybutyric acid (EP 133,988). Suitable microcapsules can also include hydroxymethyl cellulose or gelatin-microcapsules and poly-methylmethacylate microcapsules prepared by coacervation techniques or by interfacial polymerization. In addition, micro emulsions or colloidal drug delivery systems such as liposomes and albumin microspheres, may also be used. See Remington's Pharmaceutical Sciences (1990) (18th ed., Mack Pub. Co., Eaton, Pennsylvania).

The compositions of the invention, comprising the pharmaceutically active agent and a succinate compound, can be stored for extended periods of time while maintaining the physical and biological integrity of the pharmaceutically active agent. Storage can be in liquid form or as a dried formulation, which can be reconstituted by adding liquid. When pharmaceutically active agents are proteins, storage can be facilitated by drying processes, such as lyophilization. Accordingly, the protein can be stored in the form of a freeze-dried composition. Thus, in one embodiment, the invention provides a lyophilized pharmaceutical composition comprising a succinate compound and the pharmaceutically active agent.

The range of temperature at which storage of a liquid preparation of a pharmaceutically active agent and succinate is possible is 2°C to 8°C, with an expected storage life of 18-24 months or longer. The range of temperature at which storage of a dry preparation of a pharmaceutically active agent and succinate is possible is 2°C to 30°C, with an expected storage life of 18 to 24 months or longer. Formulations can be stored, however, up to 5 years. A further range for the dried formulation is 22°C to 30°C. Preferred temperatures include, but are not limited to, 2°C to 8°C for liquid, and room temperature (i.e., 25°C - 30°C) for dry. The liquid and dried formulations are stable for about 18-24 months or longer. The specific disclosed rhIGF-I formulation has been shown to be stable for at least a year at 2-8°C in liquid form.

The pharmaceutical composition is preferably sterilized by membrane filtration and is stored in unit-dose or multi-dose containers such as sealed vials or ampules. In one embodiment of the invention, the head space of the vials can be flushed with nitrogen when filling. The invention also encompasses devices for dosing convenience, such as pre-filled syringes, autoinjectors, blister packs, or needle-less systems, to make the administration or injection easier.

A pharmaceutically effective amount of the composition of the invention is administered to a subject. By pharmaceutically effective amount is meant an amount that is useful in the treatment, prevention or diagnosis of a disease or condition.

By the term "administer" is intended any suitable method for delivering a pharmaceutically active agent to a subject, including parenteral, intranasal, intrapulmonar, oral, topical, anal or surgical implantation or insertion. Typical parenteral routes of administration include, intravenous, intramuscular, subcutaneous, intraarterial and intraperitoneal injection or infusion. Preferably the administration is by injection. In preferred embodiments, injection is subcutaneous. Injectable forms of the compositions of the invention include, but are not limited to, solutions, suspensions and emulsions.

By "subject" is intended any animal. Preferably the subject is mammalian, must preferably the subject is human. Mammals of particular importance other than human include, dogs, cats, cows, horses, sheep and pigs.

When administration is for the purpose of treatment, administration may be for either a prophylactic or therapeutic purpose. When provided prophylactically, the substance is provided in advance of any symptom. The prophylactic administration of the substance serves to prevent or attenuate any subsequent symptom. When provided therapeutically, the substance is provided at (or shortly after) the onset of a symptom. The therapeutic administration of the substance serves to attenuate any actual symptom.

The pharmaceutical composition comprising reconstituted IGF-I should be formulated in a unit dosage and in an injectable or infusible form such as solution, suspension, or emulsion. It can also be in the form of lyophilized powder, which can be converted into solution, suspension, or emulsion before administration. The pharmaceutical composition comprising reconstituted IGF-I is preferably sterilized by membrane filtration and is stored in unit-dose or multi-dose containers such as sealed vials or ampules.

A pharmaceutically acceptable carrier should be mixed with the pharmaceutically active agent and the succinate buffer.. By "pharmaceutically acceptable carrier" is intended a carrier that is conventionally used in the art to facilitate the storage, administration, and/or the healing effect of the therapeutic ingredients. A carrier may also reduce any undesirable side effects of the IGF-I. A suitable carrier should be stable, *i.e*., incapable of reacting with other ingredients in the formulation. It should not produce significant local or systemic adverse effect in recipients at the dosages and concentrations employed for treatment. Such carriers are generally known in the art. Suitable carriers for this invention are those conventionally used large stable macromolecules such as albumin, gelatin, collagen, polysaccharide, monosaccharides, polyvinyl-pyrrolidone, polylactic acid, polyglycolic acid, polymeric amino acids, fixed oils, ethyl oleate, liposomes, glucose, sucrose, lactose, mannose, dextrose, dextran, cellulose, mannitol, sorbitol, polyethylene glycol (PEG), and the like. Slow-release carriers, such as hyaluronic acid, may also be suitable. See particularly Prisell et al. (1992) Int. J. Pharmaceu. 85:51-56, and U.S. Patent No. 5,166,331. Inclusion of hyaluronic acid and other polymers may have an additional beneficial effect on the IGF-I-responsive disorder osteoarthritis. See particularly Bragantini (1987) Clin. Trials J. 24(4):333-340; Dougados et al. (1993) Osteoarthritis and Cartilage 1:97-103; and Lussier et al. (1996) J. Rheum. 23:1579-1585.

The pharmaceutical composition may additionally comprise a solubilizing agent or so-called solubility enhancer. Compounds containing a guanidinium group, most preferably arginine, are suitable solubility enhancers.

The method for formulating a pharmaceutical composition is generally known in the art. A thorough discussion of formulation and selection of pharmaceutically acceptable carriers, stabilizers, and isomolytes can be found in Remington's Pharmaceutical Sciences (18th ed.; Mack Pub. Co.: Eaton, Pennsylvania, 1990).

The pharmaceutical compositions comprising IGF-I are useful in therapy directed to treatment of IGF-I responsive conditions. By "IGF-1-responsive condition" is intended any condition that responds in the short-term or in the long-term either positively or negatively to IGF-I. Such IGF-I-responsive conditions may be a normal condition. For example, a mammal may undergo IGF-I therapy to increase normal muscle mass where greater muscle mass is desirable, as in an athlete. In contrast, the IGF-I responsive condition may be an abnormal condition that is chronic, and thus occurs more or less continuously, or that is acute, as occurs following injury to a site, such as a joint or bone injury.

Conditions responsive to IGF-I include acute or chronic conditions including, but not limited to, hyperglycemic disorders, including all forms of diabetes; chronic lung disease; acute and chronic renal disorders; acute and chronic liver failure; hepatic cirrhosis; inflammatory responses, such as rheumatoid arthritis, psoriatic arthritis, Reiter's syndrome, and inflammatory bowel disease; short gut; ischemic injuries involving the heart, liver, or brain, or such as results from renal tubular necrosis; immunological disorders, such as immunodeficiencies including decreased immune tolerance or chemotherapy-induced tissue damage; organ rejection after transplantation; diseases or insufficiencies of cardiac structure or function, such as chronic heart conditions, cardiomyopathy, stroke, and congestive heart failure; growth retardation; osteoporosis; wound healing; bone damage; ophthalmic conditions; infertility; neurodegenerative disorders, such as motoneuron disease, multiple sclerosis, muscular dystrophy, diabetic neuropathy, demyelinating peripheral neuropathies, Parkinson's disease, Alzheimer's disease, and a sequela of traumatic spinal cord lesions; and articular cartilage disorders, such as osteoarthritis and trauma-related injuries. Any IGF-1-responsive disorder may benefit from administration of the pharmaceutical compositions comprising the IGF-I syrup or reconstituted IGF-I obtained therefrom of the present invention.

By "treatment" is intended treatment of an existing normal condition that is enhanced by the pharmaceutically active agent, therapeutic treatment of an existing abnormal condition, and preventive or prophylactic procedures performed before the occurrence of an abnormal disorder.

The pharmaceutical compositions of the invention may be used for the treatment of any mammal. Exemplary mammals include, cats, dogs, horses, cows, sheep, pigs, and more preferably humans.

### EXAMPLES

### Methods

IGF-I for use in these experiments was recombinantly produced in the yeast strain *Pichia pastoris* and purified essentially as described in U.S. Patent Nos. 5,324,639, 5,324,660, and 5,650,496 and International Publication No. WO 96/40776.

### Reverse Phase HPLC

The separation and quantitation of rhIGF-I species were accomplished with Cyano-Reverse Phase High Performance Liquid Chromatography (CN-RP-HPLC). Separations were accomplished on a Zorbax 300SB-CN, 4.6 mm ID x 15 cm, 5µ, cyano column with sample detection at 214 nm. Samples were diluted in water to a common concentration of 0.8 mg/ml and injection volumes were 20 l (~16 µg protein per injection). Elution was achieved with a gradient of acetonitrile/water/0.2% trifluoroacetic acid (TFA) from approximately 25% acetonitrile (ACN) to 34% ACN over 25 minutes.

The CN-RP-HPLC profile of rhIGF-I contains peaks representing "authentic" rhIGF-I, which is fully active, and several other peaks containing minor rhIGF-I species. These minor species are variants of "authentic" rhIGF-I which contain small chemical changes to the protein molecule (e.g., oxidized methionine residues, glycosylation, etc.) At Time 0, the chromatographic peak corresponding to "authentic" rhIGF-I comprises approximately 95% of the total area of all peaks. This peak, containing "authentic" rhIGF-I is referred to as the "Main Peak." To estimate the kinetics of rhIGF-I degradation by CN-RP-HPLC in stability studies, the Main Peak area percent is followed as a function of time under a given set of storage conditions. As rhIGF-I degrades, the Main Peak area percent is found to decrease as the area percent of peaks corresponding to rhIGF-I breakdown products is found to increase.

### Non-reducing SDS-PAGE

Covalent aggregates of rhIGF-I, which occur due to formation of intermolecular disulfides, can be detected on non-reducing SDS-PAGE gels. For this analysis, 18% Tris-Glycine non-reducing SDS-PAGE was run. Gels were run at constant voltage and stained with the Colloidal Coomassie. Destained gels were scanned with a densitometer and bands were converted to peak areas.

At Time 0, rhIGF-I comprises a single monomeric band on non-reducing SDS-PAGE. This band (i.e., the Monomer band) comprises 100% of the total peak area at Time 0. To determine the kinetics of rhIGF-I degradation by non-reducing SDS-PAGE in stability studies, the monomer area percent is followed as a function of time under a given set of storage conditions. As rhIGF-I degrades, the Monomer area percent is found to decrease and the area percent of other bands corresponding to rhIGF-I breakdown products (e.g., dimers, trimers, etc.) is found to increase.

### Mitogenic Bioassay

The bioactivities of rhIGF-I samples were determined using a mitogenic assay according to the procedure of W. Lopaczynski et al. (1993) Regulatory Peptides, 48:207-216. This assay is based on the dose dependent induction of a cell proliferation by rhIGF-I. The response is measured with MTT (3-[4,5-dimethylthiazol-2-yl] - 2,5 diphenyltetrazolium bromide) stain, which is reduced to a colored product by the mitochondrial enzymes of MG-63 live cells (American Type Culture Collection (ATCC CRL 1427)). The assay has been standardized against a WHO reference to provide the activity of rhIGF-I in International Units (IU). The % activity of rhIGF-I is determined in stability studies by comparison of the sample activity to a reference standard of known activity.

### Example 1

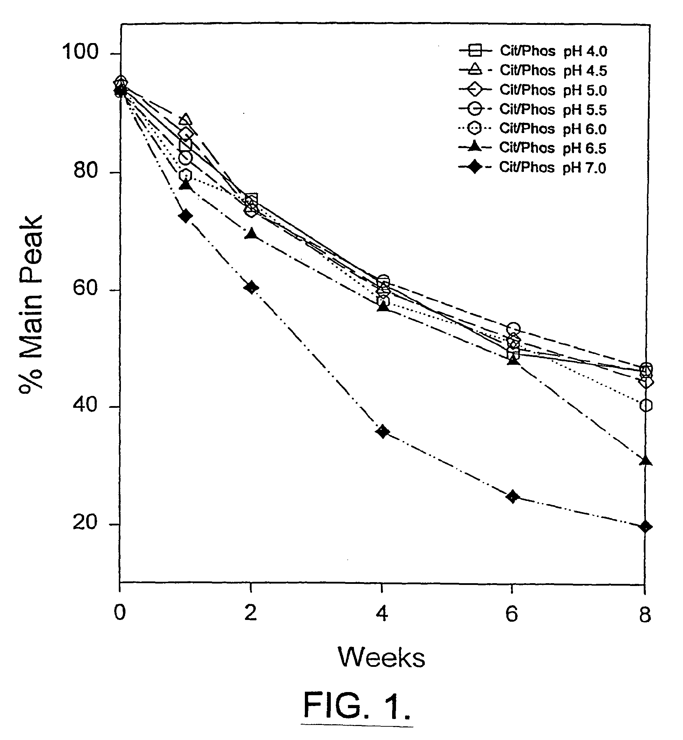

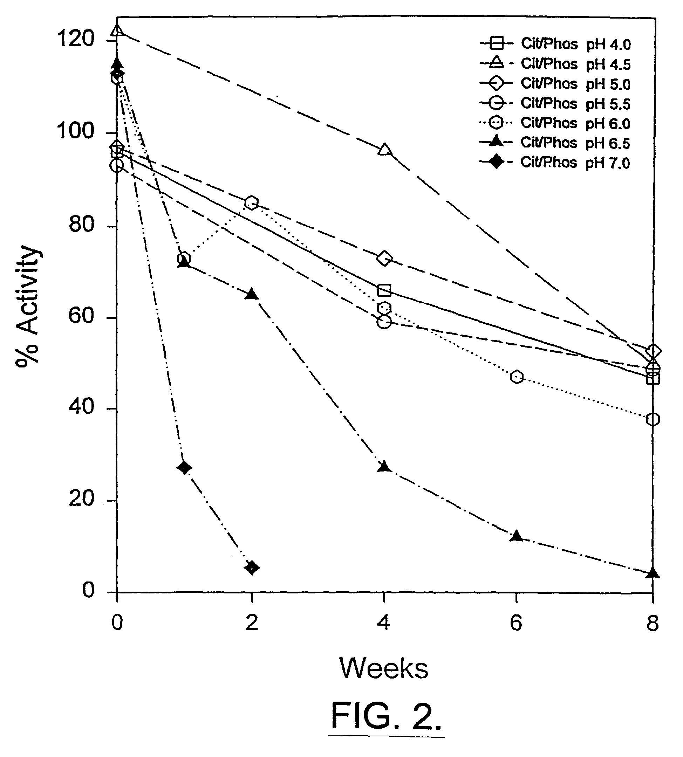

This example illustrates the stability of rhIGF-I as a function of formulation pH. For Figure 1, citrate-phosphate buffer at a pH range of 4.0-7.0 was formulated with rhIGF-I. Percent main peak (peak that contains the native molecule) integrity was measured over a period of eight weeks at a temperature of 50°C.
Measurement was by CN (cyano)-RP-HPLC. For Figure 2, citrate-phosphate buffer at a pH range of 4-7 was formulated with rhIGF. Percent activity was measured over a period of eight weeks at a temperature of 50°C. Measurement of activity was by mitogenic bioassay. The results of Example 1 indicate that pH 6.0 allows the maintenance of adequate stability of rhIGF-I in a pharmaceutical formulation.

### Example 2

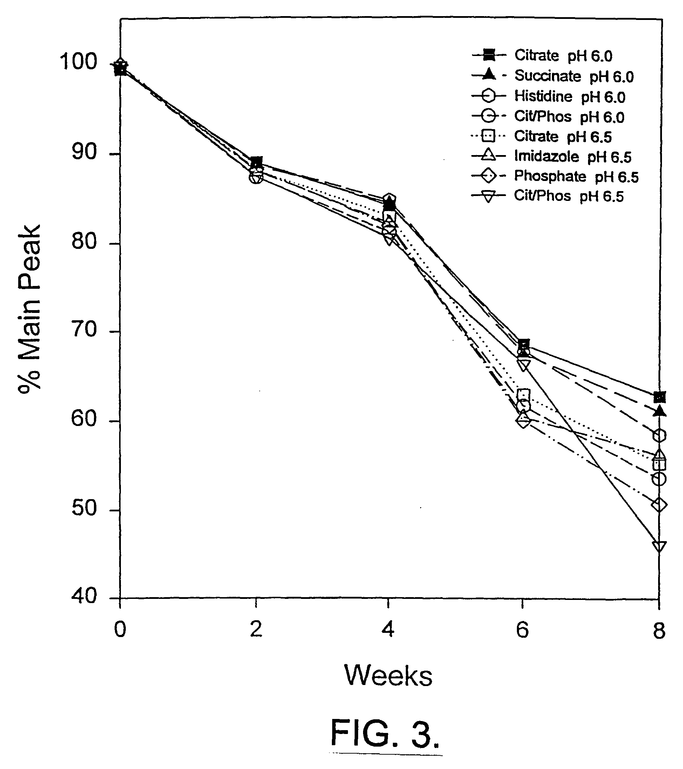

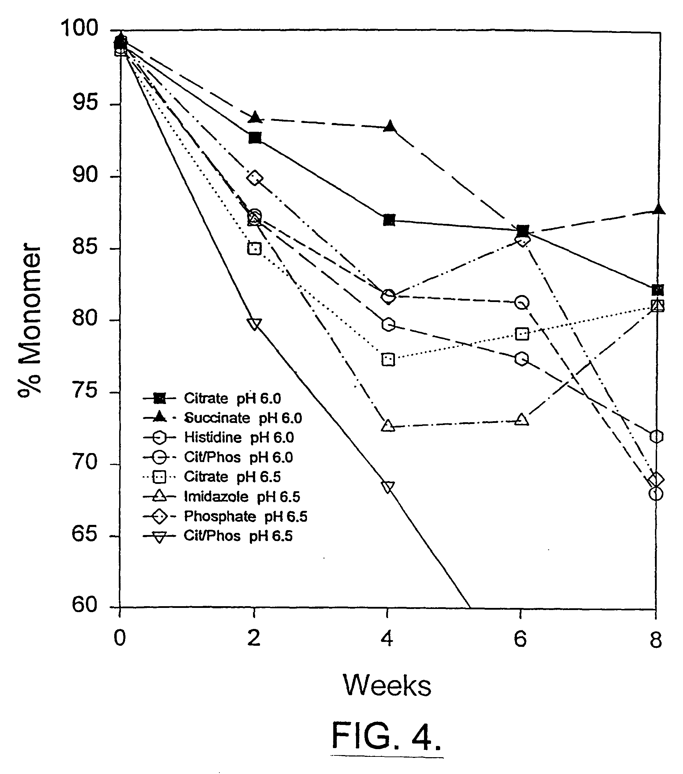

This example illustrates the stability of rhIGF at pH 6.0 and pH 6.5 as a function of buffer species. For Figure 3, various buffers were formulated with rhIGF-I. Main peak integrity was measured over a period of eight weeks at a temperature of 50°C. The integrity was measured by CN-RP-HPLC. For Figure 4, the measurement of percent monomer (native molecule) remaining was over a period of eight weeks at a temperature of 50°C. The percent monomer remaining was measured by non-reducing SDS-PAGE. The results shown in this example indicate that (1) pH 6.0 formulations provide greater stability than pH 6.5 formulations; and (2) sodium citrate and sodium succinate buffers are highly compatible with rhIGF-I at pH 6.0.

### Example 3

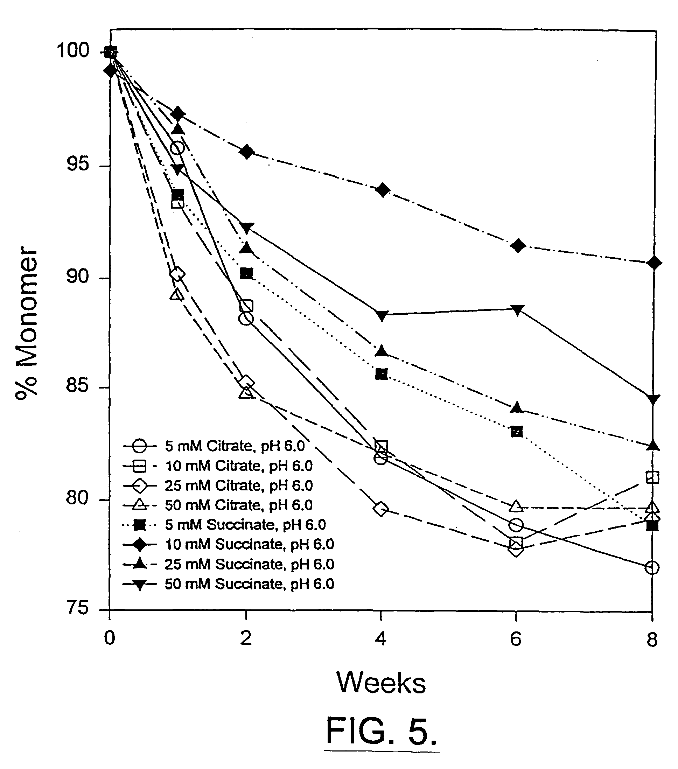

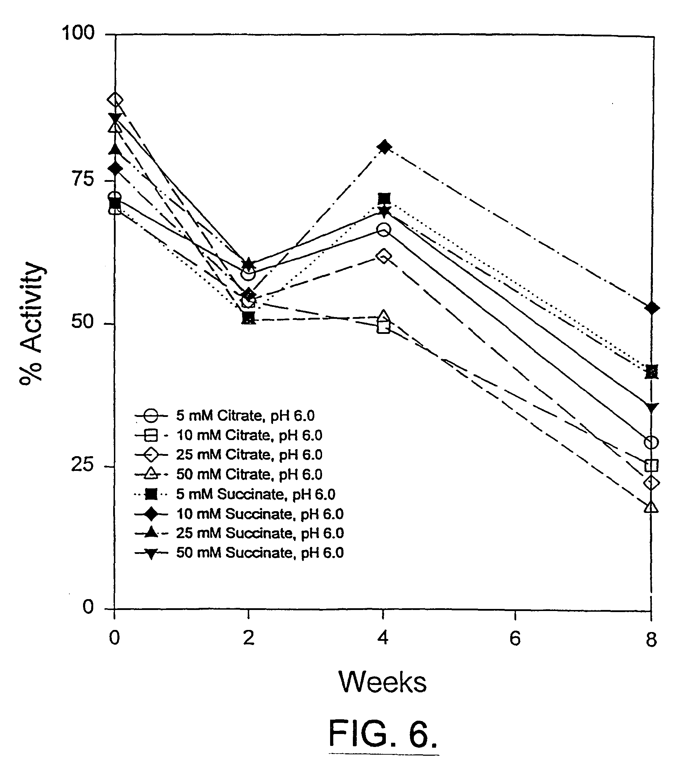

This example illustrates the stability of rhIGF-I as a function of sodium citrate and sodium succinate concentration at pH 6.0. For Figure 5, stability was measured over a period of eight weeks at 50°C. Measurement was done by assaying the percent monomer by non-reducing SDS-PAGE. For Figure 6, stability was measured by assay of activity over a period of eight weeks at 50°C. Measurement of activity was done by mitogenic assay. The result in this example indicate that succinate buffers are more compatible with rhIGF-I than citrate buffers, and that a concentration of 10 mM is optimal for rhIGF-I stability.

### Example 4

This example shows data from a nociceptor activation model designed to predict the pain of injection produced by formulations. In this model, capsaicin (a compound known to cause pain on injection) is used to generate a standard curve. Formulations were then tested and given a value based on the standard curve. All formulations were then compared to normal saline. Two buffers were tested and compared to normal saline: 0.1M sodium acetate, pH 4.0; and 10 mM sodium succinate, 140 mM sodium chloride, pH 6.0. The acetate formulation generated a response off the standard curve indicating significant injection pain. The 10 mM succinate formulation was found to be no more painful than normal saline.

### Example 5

The study described in this example was performed to assess the potential local irritant effects of pharmaceutical formulations in New Zealand White rabbits when administered by subcutaneous injections for seven consecutive days. Test articles (buffer plus IGF) vehicle controls (buffer alone), and saline were given to each rabbit at three different sites. The rabbits were administered 0.5 ml of the appropriate saline/vehicle/test article, in each of the designated test sites, once daily for seven consecutive days via subcutaneous injection. Test sites were examined for signs of erythema and edema and the sign scored prior to dosing each day and on days eight and nine according to a grading system based on Draize (Appraisal of the Safety of Chemicals in Food, Drugs, and Cosmetics (1959), the Association of Food and Drug Officials of the United States, pp. 49-51). All the injection sites and any gross lesions were trimmed and processed to paraffin blocks. The tissue sections were stained with hematoxylin and eosin and examined microscopically by a board-certified veterinary pathologist. Histological processing was performed by HistoTechnics, Powell, Ohio. The results of the histopathology indicate that saline and vehicle control injection sites exhibited relatively mild inflammation and were comparable with the exception of one saline control site with severe inflammation. The test article injection sites exhibiting the most inflammation occurred in test articles C and D. The remaining test articles were not remarkably different from each other, but caused slightly greater inflammation than the saline and vehicle control articles. The vehicle controls A, B, D, E, and F, were comparable to saline. Accordingly, while mild external dermal irritation was noted on several sites sporadically throughout the study, it was not necessarily an indication of the resulting histopathology. With emphasis on the histopathology at the injection site, test articles C and D were considered to be outstanding, with only moderate inflammation as compared to the remaining test articles, which exhibited only slightly greater inflammation over their respective saline/vehicle controls.

The following formulations were used:
(1) Saline was used as a 0.9% solution;
(2) Test articles included the vehicle buffer (below) plus 8.0 mg/ml rhIGF-I; and
(3) Vehicle Control A - 10 mM sodium succinate, 140 mM sodium chloride, pH 6.0;
   Vehicle Control B - 10 mM sodium citrate, 135 mM sodium chloride, pH 6.0;
   Vehicle Control C - O.1M sodium acetate, 50 mM sodium chloride;
   Vehicle Control D- 1 mM methionine, 135 mM sodium chloride, pH 6.0;
   Vehicle Control E- 10 mM sodium succinate, 125 mM arginine, 20 mM sodium chloride, pH 6.0; and
   Vehicle Control F - 7.4 mM ammonium citrate, 1.9% sucrose, 97 mM sodium chloride, pH 4.8.

The results are shown in Figure 8. The results show that the succinate (test article A) provided an effective medium for subcutaneous injection.

## Claims

1. An injectable pharmaceutical composition comprising human insulin-like growth factor 1 (IGF-1) or a biologically active variant thereof and a buffer, wherein said buffer consists substantially of succinate at a concentration of 10mM to 40mM and a counterion, and wherein said variant is a polypeptide having IGF-1 activity and at least 70% amino acid sequence identity to the amino acid sequence for human IGF-1.

2. The composition of claim 1, wherein said counterion is selected from sodium, potassium, ammonium, and said IGF-1.

3. The composition of claim 1 or 2, wherein the concentration of succinate is 10mM to 30mM.

4. The composition of claim 3, wherein the concentration of succinate is 10mM to 20mM.

5. The composition of claim 4, wherein the concentration of succinate is about 10mM.

6. The composition of any one of the preceding claims, wherein said composition has a pH of 4.0 to 7.0.

7. The composition of claim 6, wherein said pH is 4.6 to 6.6.

8. The composition of claim 7, wherein said pH is about 6.0.

9. The composition of any one of the preceding claims, further comprising a sufficient concentration of at least one tonicifying agent such that the composition is isotonic.

10. The composition of claim 9, wherein said tonicifying agent is sodium chloride.

11. The composition of any one of the preceding claims, wherein said human IGF-1 is recombinant human IGF-1.

12. The composition of claim 1 or 11, wherein said composition has a pH of about 6.0, the concentration of said succinate is about 10mM, and the composition further comprises about 140mM sodium chloride.

13. The composition of any one of the preceding claims, wherein said composition is a liquid.

14. The composition of any one of claims 1 to 12, wherein said composition is lyophilised.

## Patentansprüche

1. Injizierbare pharmazeutische Zusammensetzung, umfassend humanen Insulin-ähnlichen Wachstumsfaktor 1 (IGF-1) oder eine biologisch aktive Variante davon und einen Puffer, wobei der Puffer im Wesentlichen aus Succinat in einer Konzentration von 10 mM bis 40 mM und einem Gegenion besteht, und wobei die Variante ein Polypeptid mit IGF-1-Aktivität und wenigstens 70% Aminosäuresequenzidentität zur Aminosäuresequenz für humanes IGF-1 ist.

2. Zusammensetzung nach Anspruch 1, wobei das Gegenion ausgewählt ist auch Natrium, Kalium, Ammonium und dem IGF-1.

3. Zusammensetzung nach Anspruch 1 oder 2, wobei die Succinatkonzentration 10 mM bis 30 mM ist.

4. Zusammensetzung nach Anspruch 3, wobei die Succinatkonzentration 10 mM bis 20 mM ist.

5. Zusammensetzung nach Anspruch 4, wobei die Succinatkonzentration etwa 10 mM ist.

6. Zusammensetzung nach einem der vorstehenden Ansprüche, wobei die Zusammensetzung einen pH von 4,0 bis 7,0 hat.

7. Zusammensetzung nach Anspruch 6, wobei der pH 4,6 bis 6,6 ist.

8. Zusammensetzung nach Anspruch 7, wobei der pH etwa 6,0 ist.

9. Zusammensetzung nach einem der vorstehenden Ansprüche, ferner umfassend eine hinreichende Konzentration an wenigstens einem tonisierenden Mittel, sodass die Zusammensetzung isotonisch ist.

10. Zusammensetzung nach Anspruch 9, wobei das tonisierende Mittel Natriumchlorid ist.

11. Zusammensetzung nach einem der vorstehenden Ansprüche, wobei das humane IGF-1 rekombinantes humanes IGF-1 ist.

12. Zusammensetzung nach Anspruch 1 oder 11, wobei die Zusammensetzung einen pH von etwa 6,0 hat, die Konzentration des Succinats etwa 10 mM ist und die Zusammensetzung ferner etwa 140 mM Natriumchlorid umfasst.

13. Zusammensetzung nach einem der vorstehenden Ansprüche, wobei die Zusammensetzung eine Flüssigkeit ist.

14. Zusammensetzung nach einem der Ansprüche 1 bis 12, wobei die Zusammensetzung lyophilisiert ist.

## Revendications

1. Composition pharmaceutique injectable comprenant le facteur de croissance de type insuline humaine 1 (IGF-1) ou son variant biologiquement actif et un tampon, dans laquelle ledit tampon consiste essentiellement en du succinate à une concentration comprise entre 10 mM et 40 mM et en un contre-ion, et dans laquelle ledit variant est un polypeptide possédant une activité IGF-1 et au moins 70% d'identité de séquence d'acides aminés avec la séquence d'acides aminés de l'IGF-1 humain.

2. Composition selon la revendication 1, dans laquelle ledit contre-ion est choisi parmi le sodium, le potassium, l'ammonium, et ledit IGF-1.

3. Composition selon la revendication 1 ou 2, dans laquelle la concentration en succinate est comprise entre 10 mM et 30 mM.

4. Composition selon la revendication 3, dans laquelle la concentration en succinate est comprise entre 10 mM et 20 mM.

5. Composition selon la revendication 4, dans laquelle la concentration en succinate est d'environ 10 mM.

6. Composition selon l'une quelconque des revendications précédentes, dans laquelle ladite composition dispose d'un pH de 4,0 à 7,0.

7. Composition selon la revendication 6, dans laquelle ledit pH est compris entre 4,6 et6,6.

8. Composition selon la revendication 7, dans laquelle ledit pH est d'environ 6.

9. Composition selon l'une quelconque des revendications précédentes, comprenant en outre une concentration suffisante d'au moins un agent tonifiant de sorte que la composition soit isotonique.

10. Composition selon la revendication 9, dans laquelle ledit agent tonifiant est du chlorure de sodium.

11. Composition selon l'une quelconque des revendications précédentes, dans laquelle ledit IGF-1 humain est un IGF-1 humain recombinant.

12. Composition selon la revendication 1 ou 11, dans laquelle ladite composition a un pH d'environ 6, la concentration dudit succinate est d'environ 10 mM, et la composition comprend en outre environ 140 mM de chlorure de sodium.

13. Composition selon l'une quelconque des revendications précédentes, dans laquelle ladite composition est un liquide.

14. Composition selon l'une quelconque des revendications 1 à 12, dans laquelle la composition est lyophilisée.
